# EUROPEAN PATENT APPLICATION

(11) **EP 4 417 737 A1**
(43) Date of publication of application: **21.08.2024**
(21) Application number: 23157420.3
(22) Date of filing: 17.02.2023
(51) Int. Cl.: C25B 1/50, C25B 3/20, C25B 15/023, G01N 27/26, G01N 33/00, G01N 35/02, B01L 3/00, G01N 27/28, G01N 35/00

(54) **POTENTIAL-CONTROLLED MULTISENSING ELECTRONIC MICROTITER PLATE**

(71) Applicant: Methanology AG, 8212 Neuhausen am Rheinfall (CH)
(72) Inventor: HERZ, Christoph, 8212 Neuhausen am Rheinfall (CH); MEIER, Gerhard T., 8212 Neuhausen am Rheinfall (CH); MEIER, Marvin, 8212 Neuhausen am Rheinfall (CH); ORTMANN, Sebastian, 8212 Neuhausen am Rheinfall (CH)
(74) Representative: Moré, Solveig Helga

(57) **Abstract**

The present invention is directed to devices and methods for parallel evaluation of a plurality of electrochemical reactions. In particular, the present invention discloses a device (1) for cell-individual electrical and gas-analytical evaluation of at least one electrochemical reaction. The device (1) has a modular configuration and comprises a base element (3) suitable for accommodating a microtiter plate (5) comprising a plurality of wells (5a), a first support plate (7), a second support plate (11), each with electrodes (9) and counter-electrodes (13), respectively, a printed circuit board (15) electrically contacting the electrodes (9) and the counter-electrodes (13), a plurality of gas ducts (18) connected to gas sensors (24), a gas pump (26), and a lid (19), wherein lower portions of the electrodes (9) and the counter-electrodes (13) project into the wells (5a) of the microtiter plate (5). The device (1) may further comprise a control unit (28).

## Description

The present invention is directed to devices and methods for parallel evaluation of a plurality of electrochemical reactions. In particular, the present invention discloses a device for cell-individual electrical and gas-analytical evaluation of at least one electrochemical reaction. The device has a modular configuration and comprises a base element suitable for accommodating a microtiter plate comprising a plurality of wells, a first support plate, a second support plate, each with electrodes, a printed circuit board electrically contacting the electrodes, a plurality of gas ducts optionally connected to gas sensors, a gas pump and a lid. The invention thus provides a fully integrated measurement, control, regulation and evaluation system. It can independently fulfill all the tasks set and does not require any external support systems. It can be used autonomously. It typically has connections for power supply and data exchange.

Bioelectrocatalysis represents a promising alternative to modern biomanufacturing technologies. It utilizes materials from biological systems as catalysts to promote redox reactions that occur at an electrode during electrochemical reactions. Bioelectrocatalysis constitutes an attractive compromise between biocatalysis and electrocatalysis and allows for green, sustainable and efficient production of chemicals, biofuels and degradable materials (Chen et al., 2020a, The progress and outlook of bioelectrocatalysis for the production of chemicals, fuels and materials. Nature Catalysis 3, 225-244).

As the name implies, bioelectrocatalysis relies on the activity of specific biological catalysts, referred to as bioelectrocatalysts, which are capable of catalyzing reduction-oxidation (redox-) reactions and transport one or more electrons between two substrates. Typical bioelectrocatalysts known in the art include so-called electroactive microbial cells, which are equipped with electrically conductive pili (E-pili) and conductive membrane structures. In addition, particular cell organelles such as mitochondria or chloroplasts may also catalyze electrochemical reactions. However, the simplest and most commonly studied catalysts for use in bioelectrocatalytic reactions are isolated enzymes, in particular enzymes of the family of the oxidoreductases.

The action of such bioelectrocatalysts relies on specialized electron mediators such as nicotinamide adenine dinucleotide (NAD(H)) and its phosphorylated form (NADP(H)) or enzyme-bound cofactors such as flavin mono-nucleotide (FMN), flavin adenine dinucleotide (FAD), haem or other metal cofactors (Chen et al., 2020a). However, these electron mediators and cofactors have to be regenerated after each successful electron transfer reaction, which makes their use on industrial scale costly and time-consuming. To avoid the use of cofactors, one approach is to bioelectrocatalytically control the desired redox-reactions by coupling the necessary catalysts for catalyzing the reaction to an electrode. The electrode is able to substitute for one of the abovementioned electron mediators or cofactors and can act as either an electron donor or an electron acceptor to assist in the oxidation or reduction of a given substrate (Chen et al., 2020b, Fundamentals, Applications, and Future Directions of Bioelectrocatalysis, Chem. Rev 120(23), 12903-12993).

A critical requirement that must be met by any bioelectrocatalytic system is high electron transfer efficiency. Efficient electron transfer is crucial for generating high current and power density in bioelectrocatalytic systems and high space-time yield of bioelectrosynthetic systems (Chen et al., 2020b).

It is therefore important to test and verify the electron transfer efficiency of, e.g., new enzymes with a potential for being effective bioelectrocatalysts. In addition, it is necessary to determine the optimal reaction conditions for a given bioelectrocatalyst. For this purpose, the bioelectrocatalysts must be subjected to a systematic evaluation in which the catalyzed electrochemical or bioelectrochemical reactions are closely monitored.

However, the accurate analysis and evaluation of bioelectrocatalytic processes can be costly and time-consuming. Therefore, there is a need for new devices and methods that allow an uncompromised evaluation of a plurality of simultaneous electrochemical or bioelectrochemical reactions. The device and method should preferably enable the researcher to analyze and, if necessary, influence each of the monitored reactions individually.

The problem is solved by the present invention, in particular by the subject matter of the claims. The invention provides a device for cell-individual electrical and gas-analytical evaluation of at least one electrochemical reaction, comprising:
a) a base element suitable for accommodating a microtiter plate comprising a plurality of wells, wherein each well defines a distinct individual cell when being filled with an electrically conductive solution;
b) a first support plate on top of the base element, wherein the first support plate comprises a plurality of electrodes corresponding to the number and arrangement of the plurality of wells of the microtiter plate, wherein the plurality of electrodes is fixed to the first support plate in a manner that allows for electrically contacting the electrodes, wherein each electrode is configured to extend from the first support plate into the electrically conductive solution in a corresponding well in said microtiter plate, wherein the first support plate further comprises a plurality of through-holes, each through-hole being adjacent to each electrode;
c) a second support plate on top of said first support plate, wherein said second support plate comprises a plurality of counter-electrodes, wherein the number and arrangement of the plurality of counter-electrodes corresponds to the number and arrangement of the plurality of electrodes of the first support plate, wherein the plurality of counter-electrodes is fixed to the second support plate in a manner that allows for electrically contacting the counter-electrodes and wherein each counter-electrode is configured to extend from the second support plate through a corresponding through-hole in the first support plate to run adjacent to a corresponding electrode into the electrically conductive solution of a corresponding well in said microtiter plate;
d) one or more printed circuit boards (PCBs) electrically contacting each electrode of the plurality of electrodes and each counter-electrode of the plurality of counter-electrodes;
e) a gas pump configured to transport reaction gases generated during the at least one electrochemical reaction from the wells of the microtiter plate through a plurality of gas ducts inside the device to a plurality of internal or external gas sensors, wherein each individual well of the microtiter plate is further connected to a ventilation duct through which a constant flow of air can be drawn from the environment via the gas pump; and
f) a lid,
   wherein the configuration of each pair of electrode and counter-electrode extending into each well allows for electrochemical communication inside the individual cell.

Such a device is of a relatively simple structure and is therefore easy to handle for a user or an automated system. Further, it offers a high flexibility because the modular components are easily interchangeable and separable for individual processing such as coating, cleaning or other procedural steps. In addition, the device according to the invention may be used for measuring and also for synthesis reactions. It can be used for gas analysis and for bioelectrochemical reactions in parallel, i.e. simultaneously, in a simple manner. Furthermore, the analysis, evaluation and reporting may be performed in an improved way.

The invention thus provides a fully integrated measurement, control, regulation and evaluation system. It is a multisensing system. If desired, it can independently fulfill all the tasks set and does not require any external support systems. It can thus be used autonomously. It typically has connections for power supply and data exchange. It can thus also be used in combination with external evaluation or analyzing means. If desired, external gas sensors can also be used to increase flexibility.

The present invention can be understood more readily by reference to the following detailed description, examples, drawings and claims. It is to be understood that this invention is not limited to the specific devices and/or methods disclosed unless otherwise specified, as such can, of course, vary. It is also to be understood that the terminology used herein is for the purpose of describing particular aspects only and is not intended to be limiting. Throughout the invention, the term "about" is intended to be understood as "+/- 10%". If "about" relates to a range, it refers to both lower and upper limit of the range. "A" is intended to mean "one or more", if not explicitly mentioned otherwise.

All literature cited herein is herewith fully incorporated.

The device according to the invention preferably has an essentially rectangular shape in plan view and, in the assembled state, has a top side, a bottom side opposite the top side, two opposite longitudinal sides and two opposite transverse sides. However, the device can also have a different shape as long as the shape of the device does not have an undesirable influence on its function. For example, the device can be round or triangular in plan view. The device according to the invention has a modular structure, i.e., it includes different elements or modules that can be operably arranged one above the other.

The base element, the two support plates and the lid are preferably made of a break-resistant material, e.g. a metal such as aluminium or iron, steel, or a polymer, preferably a thermoplastic or duroplastic such as polyoxymethylene (POM-H or POM-C) or polyether ether ketone (PEEK) or polytetraflouroethylene (PTFE). The individual elements of the device mentioned above do not necessarily all have to be made of the same material. For example, it is possible that the two support plates are made of, e.g., polymer, while the base element and the lid are made of metal.

The optionally moulded parts that form the base element, the two support plates and the lid are preferably produced by injection moulding. Alternatively, the individual components can also be fabricated with the help of a 3D printer.

The base element defines the bottommost part or module of the device and is configured to carry the microtiter plate. For instance, the upper surface of the base element may be designed to comprise a recess or depression that is sufficiently large to accommodate and hold the microtiter plate, i.e. the microtiter plate can be inserted into the recess or depression of the base element, preferably with a precise fit. In this way, the microtiter plate is protected from slipping or from being knocked over during use of the device. The microtiter plate can be further fixated inside the recess of the base element, e.g. with the help of adjusting screws integrated in the base element, which can clamp the microtiter plate laterally. Other designs are also conceivable for accommodating and/or fixating the microtiter plate to the base element. For example, a frame element sufficiently large to accommodate a microtiter plate can be attached to the upper surface of the base element. The frame element can also comprise adjusting screws or may optionally be configured in such a way that its size can be adjusted to the dimensions of the microtiter plate that is to be inserted, for example by rendering the side walls of the frame element movable. Alternatively, the frame element can also be integral with the base element. In another aspect, the base element may also comprise one or more clamps that can be used to clamp the microtiter plate to the upper surface of the element.

Biological cells and enzymes often work optimally at a specific temperature. Thus, e.g., to test for optimal temperatures, and/or to optimize reactions, the base element may be configured to accommodate a heating block with adjustable temperature function onto which the microtiter plate is placed. A heating block may allow to adjust the reaction temperature in the individual cells of the microtiter plate during bioelectrocatalytic reactions performed therein by heating up or cooling down the temperature of the electrically conductive solution inside the wells. The heating block may be compatible with microtiter plates of a specific type and well number, e.g. the heating block may comprise a defined number of openings into which the wells of the microtiter plate can be inserted. This is advantageous because it allows for uniform heat transfer. In addition, the heating block may be configured to allow for selective temperature adjustments for each individual cell or groups of cells of the microtiter plate. The microtiter plate may optionally provide various interchangeable adapters that are able to accommodate microtiter plates with different numbers of wells. Alternatively, the heating surface of the heating block may be smooth so that any microtiter plate can be positioned on the heating block, regardless of the number of wells of the microtiter plate.

Alternatively, if a specific temperature is desired, the device as a whole may be placed in a temperature-adjusted environment.

The bottom surface of the base element can optionally be coated or equipped with a non-slip material, such as, e.g., a thin layer or film made of rubber so that the element cannot be shifted or displaced when it stands on a smooth surface, such as, e.g., on a table or a laboratory bench. Alternatively, the base element can also be equipped with suitable holding means with which the base element can be firmly attached or fixed to a table or laboratory bench. Suitable holding means may include, e.g., a screwable clamp or other clamping means.

The microtiter plate that is to be inserted into the base element of the device according to the invention may be any suitable commercially available microtiter plate. In the context of the invention, the terms "microplate", "microtiter plate", "microwell plate", or "multiwell plate" are used interchangeable and refer to a flat plate typically made of a polymer such as polystyrene, polypropylene or polycarbonate comprising at least two separate "wells", i.e., cavities into which a defined maximum quantity of a liquid can be filled. The microtiter plate may also consist of other materials such as metal or glass. Each well of a microtiter plate can serve as an independent reaction vessel or test tube in which a separate electrochemical reaction can take place, i.e., each well defines an individual isolated cell. The maximum capacity of the individual wells, i.e. the maximum reaction volume each individual well is able to accommodate, depends on the dimensions of the microtiter plate and, in particular, on the total number of wells on the microtiter plate. In general, the more wells a microtiter plate contains, the lower the maximum capacity of each individual well. Microtiter plates can have a variety of forms, sizes and shapes. They are typically produced in standard sizes and shapes with standard arrangements of wells. A microtiter plate suitable for being inserted into the device according to the invention may typically have 6, 12, 24, 48 or 96 wells that are arranged in a rectangular matrix. The microtiter plate may also have less than 6 wells, e.g., 2 or 4 wells, or more than 96 wells, e.g., 384 wells. The base area of different microtiter plates is usually the same or nearly the same, regardless of the number of wells per plate. Therefore, the base element of the device according to the invention is preferably able to accommodate and hold a variety of microtiter plates comprising different numbers of wells. The microtiter plates are preferably disposable, i.e., they may be used only once before they are removed from the device. Alternatively, the microtiter plates may also be used multiple times before being disposed.

The first support plate comprises a plurality of electrodes that are attached to the first support plate in a manner that allows for electrically contacting said electrodes. To accommodate the electrodes, the first support plate may comprise a plurality of holes, wherein each hole extends from an opening in the bottom surface of the first support plate all the way through the first support plate to an opening in its upper surface. The electrodes can be inserted into these holes such that an upper portion of the electrodes protrudes from the upper surface and a lower portion of the electrodes protrudes from the bottom surface of the first support plate. The electrodes may be attached to the first support plate by adhering, pressing, welding, screwing, plugging or by any other suitable fixing method. Preferably, the electrodes are attached to the first support plate via plugging. The upper portion of each electrode, that is, the part of the electrode that extends upwards from the upper surface of the first support plate is freely accessible and thus can be electrically contacted.

The holes inside the first support plate are arranged in a rectangular matrix and may correspond in number and arrangement to the wells in the microtiter plate that is to be placed into the base element. Accordingly, when the first support plate is arranged on top of the base element comprising a microtiter plate, each individual electrode attached to the first support plate extends with its lower portion into a corresponding well of the microtiter plate. The lower portion of the electrodes protrude deep enough into the individual wells of the below microtiter plate so that in case the wells are filled at least to half the maximum capacity with an electrically conductive solution, the electrodes will extend into said solution.

The number of holes in the first support plate does not have to correspond to the number of wells in the microtiter plate. It may, e.g., be possible that the number of holes in the first support plate exceeds the number of wells of the microtiter plate used. In such a case, it may suffice that only a subset of these holes carry an electrode. On the other hand, it is also possible that the number of wells in the microtiter plate exceeds the number of holes in the first support plate. Consequently, in such a case, some wells of the microtiter plate have to be omitted when using the device according to the invention.

The device according to the invention may also provide a collection of different alternative first support plates, which can accommodate a different maximum number of electrodes. When assembling the device according to the invention, a suitable support plate can be selected depending on the number of wells of the microtiter plate used.

Adjacent to each hole required for holding an electrode, the first support plate exhibits another through-hole. The through-hole is configured to allow a counter-electrode connected to the second support plate on top of the first support plate to extend through the first support plate into a corresponding well of the microtiter plate below. Accordingly, the first support plate comprises a plurality of first through-holes for allowing the plurality of counter-electrodes of the second support plate, and, optionally, the gas ducts, to pass through the first support plate.

The first support plate is optionally also configured to seal the individual wells of the microtiter plate. Accordingly, the bottom surface of the first support plate may comprise a sealing layer, e.g. of rubber or latex, which presses onto the microtiter plate. In some embodiments, the sealing layer may not be part of the first support plate but constitutes a separate component or module of the device of the invention that separates the microtiter plate from the overlying support plates. In this case, the sealing layer may be, e.g., a separate mat or sheet made, e.g., of a rubber material, which comprises a plurality of holes arranged to allow the electrodes, counter electrodes and gas ducts to pass through the sealing layer into each respective well. For example, the sealing layer may comprise a number of holes equal to the number of wells in the microtiter plate to be used and having a diameter large enough to accommodate the electrodes and gas ducts for one well each. Alternatively, the sealing layer can also provide a separate passage for each electrode and for each gas duct.

The sealing layer prevents gases produced during the bioelectrocatalytic reaction inside the wells from escaping in an uncontrolled manner or substances or gases from being exchanged between adjacent wells. This ensures that each well of the microtiter plate is a self-contained and isolated cell.

The plurality of counter-electrodes are attached to a second support plate which resembles the first support plate in design. Like the first support plate, the second support plate may also exhibit a plurality of holes, wherein each hole extends from an opening in the bottom surface of the second support plate all the way through the second support plate to an opening in its upper surface. The upper portion of the counter-electrodes can be inserted into these holes such that the counter-electrodes completely penetrate the second support plate. The upper portion of each counter-electrode is therefore freely accessible on the upper surface of the second support plate and thus can be electrically contacted. Attachment of the counter-electrodes to the second support plate may be achieved via adhering, pressing, welding, screwing, plugging or by any other suitable fixing method. Preferably, the counter-electrodes are attached to the second support plate via plugging.

The holes inside the second support plate which hold the plurality of counter-electrodes are arranged in a rectangular matrix and preferably correspond in number and arrangement to the corresponding holes carrying the electrodes of the first support plate as well as to the wells in the microtiter plate that is to be placed into the base element. The holes on the second support plate into which the plurality of counter-electrodes are inserted are not positioned directly above the holes on the first support plate that carry the plurality of electrodes within the assembled device, but are slightly offset. In fact, when the device is fully assembled, the counter-electrodes are positioned directly above the through-holes of the first support plate located adjacent to each electrode. When the second support plate is arranged on top of the first support plate in the assembled device, each counter-electrode that is attached via a corresponding hole to the second support plate therefore extends with its lower portion through a corresponding through-hole of the first support plate into a corresponding well of the microtiter plate. In doing so, each counter-electrode of the second support plate combines with a corresponding electrode of the first support plate to form a pair of electrode and counter-electrode inside a common well of the microtiter plate. The electrode and the counter electrode of such a pair are not in direct contact with each other but run adjacent to each other, e.g. in parallel or nearly in parallel while being separated by a gap. Each pair of electrode and counter-electrode only electrically communicate when being in contact with an electrically conductive solution in an individual well of the microtiter plate.

Optionally, the first support plate and the second support plate are essentially interchangeable, i.e., the first support plate can be arranged either below or above the second support plate when the device according to the invention is assembled. If they are interchangeable, the electrodes may have to be vertically adjustable. Thus, they can be inserted into the wells of the microtiter plate and contact the respective electrical contacts of the control unit.

As for the first support plate, the number of holes in the second support plate does not have to correspond to the number of wells in the microtiter plate. It may, e.g., be possible that the number of holes in the second support plate exceeds the number of wells of the microtiter plate used. In such a case, it may suffice that only a subset of these holes carry a counter-electrode. On the other hand, it is also possible that the number of wells in the microtiter plate exceeds the number of holes in the second support plate. Consequently, in such a case, some wells of the microtiter plate would have to be omitted when performing an analysis analyzed using the device according to the invention.

Preferably, however, the number of holes in the second support plate corresponds to the number of holes in the first support plate, so that the total number of the plurality of electrodes and the plurality of counter-electrodes is also equal. Only then can each electrode of the first support plate form an electrode pair with a corresponding counter-electrode of the second support plate.

The device according to the invention may also provide a collection of different alternative second support plates, which can accommodate a different maximum number of counter-electrodes. When assembling the device according to the invention, a suitable support plate can be selected depending on the number of wells of the microtiter plate used.

The second support plate further comprises a through-hole adjacent to each counter-electrode for receiving the upper portion of a corresponding electrode from the first support plate. In consequence, the upper portions of both the plurality of electrodes and the plurality of counter-electrodes are freely accessible on the upper surface of the second support plate and can thus be electrically contacted.

In one embodiment, the first support plate and the second support plate can also be integrally formed. In this case, the plurality of electrodes and the plurality of counter electrodes are fixed to a common support plate. Preferably, the first support plate and the second support plate are separately formed, as this allows for more flexible handling, e.g., separate coating or cleaning of electrodes.

In one embodiment, the plurality of electrodes of the first support plate are a plurality of anodes and the plurality of counter-electrodes of the second support plate are a plurality of cathodes. In an alternative embodiment, the plurality of electrodes of the first support plate are a plurality of cathodes and the plurality of counter-electrodes of the second support plate are a plurality of anodes. This allows for a flexible structure and use of the device, and in particular a control of the reaction which can be defined as desired.

An electrode is an electrical conductor capable of making contact with a nonmetallic part of a circuit, e.g., an electrolyte. An anode represents an electrode at which an oxidation reaction occurs, i.e., the anode accepts electrons from a substrate in the environment, whereas the reduction reaction occurs at the cathode, i.e. the cathode is capable of transferring electrons to a substrate.

The plurality of electrodes and/or the plurality of counter-electrodes are preferably solid and of elongated shape. For example, the electrodes and counter-electrodes are rod - shaped and, optionally, of uniform thickness. However, the electrodes and/or counter-electrodes can also have other, typically elongated shapes, e.g. they can be spiral-shaped. Preferably, the electrodes and counter-electrodes take the shape of flat rods, i.e. elongated plate electrodes. Electrodes of such a shape are less fragile and inexpensive to produce. They are also particularly suitable for being coated. In one embodiment, the upper portions of the plurality of electrodes and/or the plurality of counter-electrodes may be of a different shape than their lower portions. For example, the upper electrode/counter-electrode portions, which are fixed to either the first or the second support plates, may be narrower or broader than the lower portion, which extends down the wells of the microtiter plate.

The plurality of electrodes and the plurality of counter-electrodes may exhibit the same dimensions, i.e., they may be of identical size and shape. Alternatively, the size and/or shape of the plurality of electrodes may be different from the size and/or shape of the plurality of counter-electrodes.

The plurality of electrodes and the plurality of counter-electrodes used in the device according to the invention preferably exhibit several or all of the following characteristics: They should preferably exhibit a biocompatible surface, an adequate specific surface area, a strong chemical and mechanical stability and a high conductivity. The electrodes and/or counter-electrodes may e.g., comprise, optionally consist of a material selected from the group comprising carbon, glassy carbon, copper, lead, zinc, tin, platinum, silver, gold, stainless steel, iron, metal alloys, conductive ceramics or conductive polymers.

The plurality of electrodes and the plurality of counter-electrodes may consist of the same or different materials.

It is even possible that individual electrodes of the plurality of electrodes consist of or comprise different materials. For example, a first fraction of the plurality of electrodes may consist of a first material such as, e.g., carbon, whereas a second fraction of the plurality of electrodes consists of a second material, such as copper. Dependent on the number of electrodes fixed to the first support plate, it may even be possible that each individual electrode of the plurality of electrodes consist of a different material. The same applies accordingly to the plurality of counter-electrodes. Thus, different electrode materials can be tested for their suitability for use in a specific reaction, e.g., with a specific enzyme.The plurality of electrodes and the plurality of counter-electrodes are preferably exchangeable. Accordingly, the electrodes or counter-electrodes can be removed collectively or individually from the first or second support plate, respectively, to be replaced e.g., with electrodes and/or counter-electrodes of a different material or completely new identical ones. Alternatively, it is also possible that the electrodes and the counter-electrodes are firmly connected to the first and the second support plate, respectively, and thus cannot be exchanged.

In addition or alternatively, the plurality of electrodes and the plurality of counter-electrodes are preferably re-usable. Accordingly, each electrode and each counter-electrode may be used in more than one subsequent experiment. For instance, the electrodes and/or counter-electrodes may be coated with a first set of enzymes to promote a first set of bioelectrochemical reactions, before being washed and coated with a second set of enzymes to promote a second set of electrochemical reactions.

Optionally, the electrodes and counter-electrodes of the device according to the invention are directly heatable. Different directly heatable electrodes, in which the heating current and the electrolysis current flow together through the same conductor, are known from the state of the art. Direct electrical heating of the electrode and simultaneously interference-free electrochemical measurement can be made possible by a so-called symmetrical arrangement or special filter circuits. A variant of the directly heated electrode has a third contact for the connection to an electrochemical measuring device exactly in the middle between the two contacts for the supply of the heating current. This arrangement prevents disturbing influences of the heating current on the measuring signals.

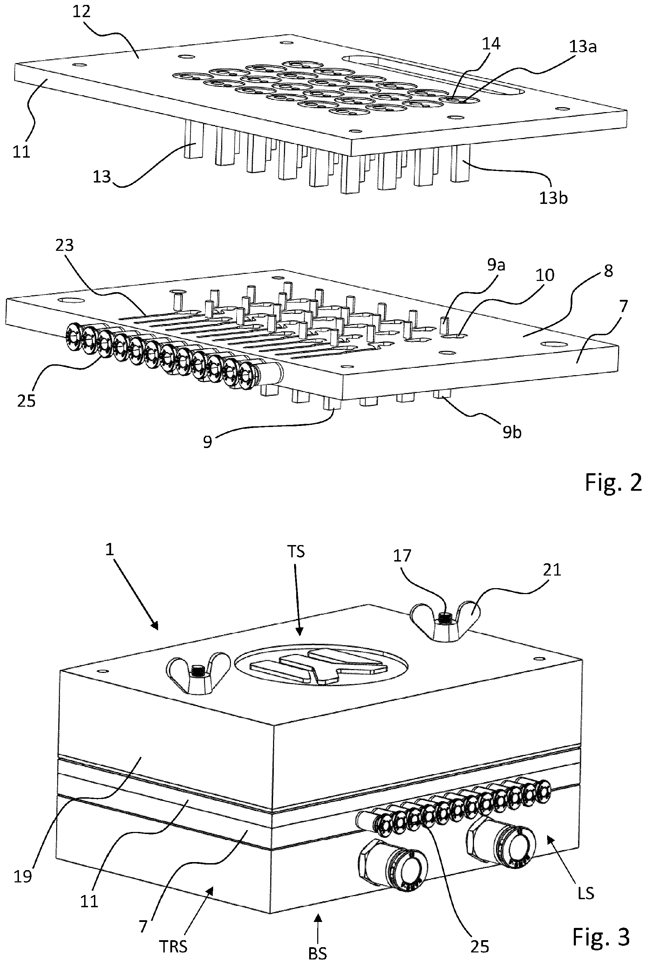

Alternatively, the electrodes and/or counter-electrodes can be heated directly by means of a symmetrical arrangement using a heating current in the form of an alternating current, whereby the use of a bridge circuit is particularly advantageous. The symmetrical contacting and direct heating of the electrodes and/or counter-electrodes can thus be achieved by means of a bridge circuit as explained, for example, in Wachholz et al., 2007, Electro-analysis 19, 535-540, in particular Fig. 3. In particular, the electrodes and/or counter-electrodes can have a first and a second connection for the supply of the heating current, the electrodes and/or counter-electrodes being connected to a potentiostat via a third connection, the connection of the third connection to the electrodes and/or counter-electrodes being formed via a bridge circuit which is connected to the first and second connection. Suitable circuits are disclosed, for example, in DE 2006 006 347.

In a preferred embodiment, each electrode and each counter electrode can be directly heated in a selective and individual manner. This allows the reaction temperature in the respective wells of the microtiter plate to be individually adjusted.

Optionally, the device according to the invention may comprise additional support plates, i.e. a third, fourth or even a fifth support plate. Each of these additional support plates may support, e.g., a plurality of additional electrodes that are attached to their assigned support plate as described herein. For example, in one embodiment, the device according to the invention may comprise a third support plate comprising a plurality of reference electrodes that are attached to the third support plate in a manner that allows for electrically contacting said reference electrodes, and that may, e.g., be linked to a potentiostat. Like the first and second support plates, the third support plate may, e.g., comprise a plurality of holes arranged in a rectangular matrix as described above for holding the reference electrodes, wherein each hole extends from an opening in the bottom surface of the third support plate all the way through the third support plate to an opening in its upper surface. The upper portion of the reference electrodes can be inserted into these holes such that the reference electrodes completely penetrate the third support plate. The upper portion of each reference electrode is therefore freely accessible on the upper surface of the third support plate and thus can be electrically contacted. Optionally, the reference electrodes may also be directly attached to the bottom surface of the one or more printed circuit boards (PCBs) described in more detail below. In such an embodiment, the composition according to the invention thus does not necessarily have to comprise an additional third support plate for carrying the reference electrodes.

When the device according to the invention is fully assembled, the lower portion of each individual reference electrode protruding from the bottom surface of the third support plate or the bottom surface of the PCB extends into the electrically conductive solution inside a corresponding well of the microtiter plate.

If the device according to the invention comprises a third support plate holding the plurality of reference electrodes, said third support plate further comprises a plurality of through-holes through which, dependent on the exact arrangement of the different support plates in the assembled device, either the upper portion or the lower portion of the plurality of electrodes and the plurality of counter-electrodes can extend, wherein each through-hole of said plurality of through-holes is located adjacent to a corresponding reference electrode.

The third support plate or the one or more PCBs carrying the plurality of reference electrodes may be arranged at any position between the base element holding the microtiter plate and the control unit or the lid. For example, the third support plate or the one or more PCB may be arranged on top of the second support plate. In such a case, the lower portions of the reference electrodes extend through corresponding through-holes in the second and the first support plate to run adjacent, e.g., in parallel or nearly in parallel to their corresponding electrodes and counter-electrodes into the electrically conductive solution of their corresponding wells of the microtiter plate. The through-holes in the first and the second support plate through which the reference electrodes extent are preferably the same through-holes through which the electrodes or counter-electrodes also pass. Alternatively, the reference electrodes may also extend through distinct through-holes in the first and second support plate as long as, ultimately, always one electrode, one counter-electrode and one reference electrode combine in a common well of the microtiter plate.

At the same time, the upper portions of the electrodes from the first support plate and the upper portions of the counter-electrodes from the second support plate extend through their corresponding through-holes in the third support plate so that they can be electrically contacted. This is, of course, not necessary in embodiments where the reference electrodes are attached directly to the PCB, thus eliminating the need for a third support plate. However, it is to be understood that the device according to the invention may also be arranged so that the optional third support plate is arranged, e.g., between the first and the second support plate or below the first support plate on top of the microtiter plate. In the latter case, the third support plate is preferably configured to seal the microtiter plate, e.g. by having its bottom surface covered with a suitable sealing layer, e.g. of rubber or latex, which presses onto the microtiter plate. The third support plate does not comprise a sealing layer in case a sealing layer is provided as a separate sheet as described herein.

The reference electrodes may be, e.g., silver electrodes, silver-silver chloride electrodes or mercury electrodes, as known to the skilled person from the prior art.

Each individual electrode, each individual counter-electrode, and optionally, each reference electrode is electrically contacted by one or more printed circuit boards (PCBs), e.g., via flexible spring contacts. The individual electrodes can be contacted single or multiple times, e.g., via double spring contacting. The latter option allows more stable contacting of the electrodes, which may compensate for potential assembly inaccuracies, manufacturing tolerances or signs of wear after prolonged use and reduces contact resistances. The one or more PCBs provide an electrical connection and are therefore able to transmit a current and/or voltage to each pair of electrodes to drive electrolysis in each individual cell of the microtiter plate. For this, the one or more PCBs are furthermore operably connected to a suitable power source such as a power plug or a battery.

The one or more PCBs can be attached at any position of the device. In a preferred embodiment however, a single PCB is mounted on top of the second support plate and is in direct contact with the upper portions of the plurality of electrodes and the plurality of counter-electrodes. In another embodiment, the PCB may also optionally be incorporated into or connected to the lid. For example, the PCB may be screwed, glued or otherwise fixed to the inner surface of the lid. In this way, the sensitive electronic components on the PCB are protected from being damaged by the user or from becoming contaminated over time.

However, the one or more PCBs can just as easily be positioned elsewhere on the device. For example, the PCB can also be positioned between the first support plate and the second support plate, between the sealing layer and the first support plate or on the side of the device. In these cases, the PCB may need to be modified accordingly. For instance, the PCB may exhibit holes to allow e.g., the plurality of counter-electrodes to protrude though empty parts of the PCB. If the PCB does not rest on the second support plate and is therefore not in direct contact with the plurality of electrodes and the plurality of counter-electrodes, it is further necessary to connect the PCB to the electrodes and/or counter-electrodes via suitable cables or wires or circuitry.

The one or more PCBs are operably connected to a suitable control unit (CU) of a central processing unit (CPU). The CU can be a part of an external computer which is not part of the device according to the invention. However, in a preferred embodiment, the device comprises a CU that is electrically connected to the one or more PCBs. Preferably, the CU is part of a single-board-computer (SBC), e.g., a microcontroller unit (MCU), that may be directly mounted onto the PCB and thus may constitute an integral part of the device according to the invention.

The CU is configured to receive and evaluate data from the electrolysis experiments performed in each well of the microtiter plate. For this, the CU is connected to a plurality of sensors including, e.g., current sensors for monitoring current-consumption and potential sensors for monitoring the applied potential of each individual cell. In some embodiments, these sensors are mounted onto the one or more PCBs, e.g., the different sensors may be mounted either onto a common PCB or, alternatively, onto different PCBs dependent on the exact set-up of the device according to the invention. The sensors may also be part of the CU, e.g., a SBC mounted onto the PCB may be configured to already comprise said sensors. The CU is capable of continuously processing, recording, evaluating, and analyzing the various signals received from the sensors. Based on the obtained information, the CU can then directly control and/or adjust the reactions performed in each individual cell of the microtiter plate by altering, e.g., the voltage applied to each individual electrode and thus the potential in each cell. Moreover, the CU is preferably connected to a suitable local IT infrastructure such as, e.g., an external computer running a suitable analysis software equipped with peripheral devices such as a monitor for displaying the data as well as one or more input devices. In some embodiments, the CU may also be operable to control the adjustable temperature function of the heating block or the direct heating of electrodes, if applicable.

Bioelectrocatalytic reactions are frequently associated with the formation of reaction gases. These gases may originate from the main reaction as well as from various side reactions taking place in each cell of the microtiter plate and may include, e.g., H₂, CO₂, CH₄ and various volatile organic compounds or compounds present in the gaseous phase under the respective reaction conditions. Thus, the device according to the invention comprises a plurality of gas ducts that enable the transport of these reaction gases from every individual cell of the microtiter plate to gas sensors. The gas sensors can be internal, i.e., the device can comprise the gas sensors, which simplifies use. Alternately, external gas sensors can be used, which can be operably connected to the gas ducts, e.g., by tubing. In this case, the gas sensors can also be used for other purposes when the device of the invention is not in use. In a preferred embodiment, the gas sensors are internal gas sensors that are, e.g.mounted onto one or more PCBs.

The gas ducts may, e.g., run through additional through-holes present in the support plates as well as through corresponding through-holes within the one or more PCBs. The gas ducts may alternatively also pass through the same through-holes of the various support plates as the plurality of electrodes and/or counter electrodes. In further embodiments, the gas ducts do not extend through all of the support plates and the one or more PCBs but run at least in part along any of the sides of the device according to the invention.

Since the amounts of reaction gases produced in each cell of the microtiter plate are expected to be small, the device according to the invention further comprises a gas pump capable of aspirating the produced gases in a uniform and controlled manner from the individual wells through the gas ducts to the gas sensors. Preferably, the gas pump should be able to pump gases both out of or into the wells of the microtiter plate. The gas pump may be positioned at any suitable location within the device of the invention. In one embodiment, the gas pump is mounted on the PCB.

The device according to the invention comprises a plurality of gas sensors which are connected to the CU. The gas ducts direct the reaction gases to said gas sensors. Suitable gas sensors for use in the device can be, e.g., Micro-Electro-Mechanical Systems (MEMS) sensors or chemical sensors such as chemiresistors, e.g., metal oxide chemiresistors, as well as solid electrolyte sensors, nondispersive infrared (NDIR) sensors or various combinations of the aforementioned sensor types. The gas sensors are preferably suitable for the detection of gas components in the ppm range, preferably in the range of 1-10,000 ppm. As with the other sensors described herein, the gas sensors transmit their signals to the CU, where they are recorded and analyzed. The CU is further connected to the gas pump in order to control its activity and thus the amount of gas that passes the gas sensors.

To avoid the development of a vacuum inside the wells of the microtiter plate upon aspiration of the reaction gases and to enable a uniform gas flow through the gas ducts past the gas sensors, each individual cell of the microtiter plate is further connected to a ventilation duct through which a constant flow of air can be drawn in from the environment by the gas pump. Alternatively, the ventilation duct may also be used in some embodiments to release gas into the environment to avoid the built-up of overpressure inside the individual wells. Several ventilation ducts can also be interconnected so that, for example, a row of adjacent wells of the microtiter plate can each be supplied with air via a joint ventilation duct. The ventilation ducts may preferably extend from the inside of each individual well of the microtiter plate though at least the first support plate where they may arrive at a single or a plurality of sealable outlets located at a surface of the first support plate. In alternative embodiments, the ventilation ducts may also extend through the second support plate and, optionally, also the printed circuit board and/or the lid before leaving the device according to the invention via outlets. The ventilation ducts preferably have a diameter of 1 mm or of less. In some embodiments, the openings of the ventilation ducts may also be connected via suitable tubing to an external gas analyzer.

The lid may be designed as a cover plate capable of covering and thus protecting the electronic components of the device from the environment. The lid may cover the first and the second support plates and can be directly connected to the base element of the device. Alternatively, the two support plates can be clamped between the lid and the base element of the assembled device. In any case, the lid is configured to be able to uniformly press the bearing surface of the elements and plates together.

The lid may comprise a preferably releasable closing mechanism which firmly connects the lid to the base element. Alternatively, the closing mechanism can be attached to the base element. For example, the closing mechanism may be one or more clamps or clasps that are fixed to, e.g., one or more sides of the base element. The one or more clamps or clasps can then be stretched over the lid to close the device. Preferably, the lid further comprises an electromagnetic shielding made of a sheet metal, a metal screen or metal foam. Suitable sheet metals for shielding are known to the skilled person and include copper, brass, nickel, silver, steel, and tin.

In another embodiment, the lid can be screwed to the base element and, optionally, to the first and second support plates and/or the PCB of the device according to the invention. For example, the base element may comprise two or more screws on, e.g., the opposite transverse sides. The threads of these screws project upwards perpendicularly to the upper surface of the base element. When the device according to the invention is assembled, the two support plates, the lid and, optionally the PCB are stacked on top of the base element by passing the threads of the at least two screws through corresponding holes drilled through the each element/plate. To fix the lid firmly on the device, nuts can be screwed tightly onto the screws protruding through the upper surface of the lid.

The stacked modules of the device according to the invention can additionally or alternatively be stabilized by means of pins which, e.g., extend from base element upwards through the other modules. In a preferred embodiment, some or all of the various modules of the device according to the invention described herein, in particular the base element, the various support plates as well as the lid comprise guides on one or more sides through which guide rods that, similar to the aforementioned screws, project upwards perpendicularly to the upper surface of the base element, are passed in order to connect the different modules of the device to each other. The guides can be designed, e.g., as pillow block bearings.

The device according to the invention can optionally be further configured to enable process-automated guiding operations, e.g. the base element can comprise one or more holders or guides for holding by an automated pipetting robot.

The device of the invention can be used for carrying out at least one electrochemical reaction, preferably, a plurality of electrochemical reactions in parallel, e.g., in each well of the microtiter plate. Advantageously, the device can be used for cell-individual electrical and/or gas-analytical evaluation of at least one electrochemical reaction. Alternatively, it can be used for electrochemical synthesis reactions.

The invention thus provides a method for carrying out at least one electrochemical reaction using the device according to the invention, wherein the method comprises steps of:
i) inserting a microtiter plate into the base element,
ii) filling at least one well of the microtiter plate with an electrically conductive solution,
iii) optionally, coating at least one of the plurality of electrodes and/or one of the plurality of counter-electrodes with an enzyme and/or a cell and/or another inorganic, organic or mixed organic/inorganic material, preferably an enzyme,
iv) assembling the device;
v) carrying out at least one electrochemical reaction, optionally at least one bioelectrochemical reaction, in the device according to the invention.

The method can be a method of preparing a product of the at least one electrochemical reaction, e.g., a reaction described in WO2016/078649 A2. The reaction has at least one, e.g., two educts, and at least one product, e.g., two products. Optionally, vi) at least one product is isolated from the wells. If the product is gaseous, e.g., H₂, the product can be isolated via the gas or ventilation ducts. The isolation can thus be carried out simultaneously with the reaction.

At least one educt of the reaction may be a component of the electrically conductive solution, or it may be added to the same before the device is assembled. Alternatively, if the educt is gaseous (e.g., H₂, CO₂ or methane), it may also be led into the microtiter plate via the ventilation duct.

When at least one bioelectrochemical reaction is to be performed using the device according to the invention, the plurality of electrodes and/or the plurality of counter-electrodes are preferably coated with an enzyme, a biological cell and/or any other inorganic, organic or mixed organic/inorganic material, preferably an enzyme. Bioelectrocatalytic reactions require the presence of specific enzymes or electroactive microbial cells as bioelectrocatalysts. These bioelectrocatalysts are capable of catalyzing the redox reactions necessary for the formation of a desired reaction product. The exact choice of bioelectrocatalyst depends on the reaction that is to be performed with the device according to the invention and the desired product that is to be obtained. For example, enzymes as disclosed in WO2016/078649 A2 may be used.

In case the electrodes and/or counter-electrodes are coated with an enzyme, the enzyme is preferably an oxidoreductase. Oxidoreductases used in bioelectrocatalysis can be classified as metalloenzymes and non-metalloenzymes, based on the cofactor normally required by said enzymes. Metalloenzymes contain metallocofactor motifs such as haem centres, iron centres, iron-sulfur clusters, copper centres, molybdenum centres or tungsten centres. The co-factors of non-metalloenzymes comprise FMN, FAD or pyrroloquinoline quinone (PQQ). The catalytic function of oxidoreductases depends on the function of their co-factors.

In case the electrodes and/or counter-electrodes are coated with a cell, said cell preferably is an electroactive microbial cell. Whole microbial cells are usually able to catalyse a wider range of reactions and usually exhibit a broader substrate specificity than isolated enzymes. Well known electroactive microbial cells that can be used as bioelectrocatalysts in the device according to the invention comprise *Geobacter sulfurreducens* or *Shewanella oneidensis.*

The electrodes and/or counterelectrodes may optionally also be coated with an organelle of a biological cell capable of catalyzing an electrochemical reaction, such as, e.g., a mitochondrion or a chloroplast.

Coating the electrodes and/or counter-electrodes with a bioelectrocatalyst as described herein allows for electron transfer between the electrode/counter-electrode and the catalyst. Dependent on the bioelectrocatalyst used, said electron transfer may further require the presence of a suitable electron transfer mediator such as methyl viologen (MV), anthraquinon-2,6-disulfonate (AQDS) or neutral red (NR). Some electroactive microbial cells produce specific metabolites themselves that can serve as natural electron transfermediators. Moreover, some enzymes do not require the presence of a mediator for electron transfer, i.e., the bioelectrocatalysts used to coat the electrode and/or counter-electrode may be capable of direct electron transfer.

The bioelectrocatalysts are preferably immobilized on the surface of the electrodes and/or the counter electrodes. Enzyme immobilization leads to better positioning of enzymes on the electrode surface and thus better electron uptake, which in turn leads to higher activities and product formation rates. On the other hand, immobilization also stabilizes the enzymes. The macromolecules are always subject to conformational changes, which contribute to a certain extent to their catalytic activity. However, external influences (e.g. temperature or radiation) can also cause irreversible conformational states, and the enzymes can aggregate, disabling the function of the biocatalyst. Immobilisations help to bind the enzymes locally in such a way that inactive conformational states are no longer assumed and prevent aggregation.

For example, an enzyme can be immobilized in alginate on a carbon fabric. Alginate immobilisation stabilises the enzymes, but their positioning in relation to the electrode is undirected.

Alternatives to alginate immobilisation for better positioning of enzymes on electrodes are, for example, immobilisation on carbon nanotubes via functional enzyme groups, on gold surfaces via covalent sulphur bridges or on Ni materials via histidine residues on the enzyme. For this purpose, corresponding amino acids could be attached to the enzymes during production, which bind directly to carrier materials of the heated electrodes.

Coating the electrodes and/or counter-electrodes with e.g., Au-, Pd- or Ni-metal layers may also reduce the activation energy threshold required to initiate electron transfer.

Alternatively, the electrodes and/or counter-electrodes may be treated with ammonia gas, chitosan, cyanuric chloride, 3-aminopropyltriethoxysilane, or melamine to improve electron transfer. Coating the electrodes and/or counter-electrodes with carbon nanotubes or graphene may result in the formation of a conductive three-dimensional matrix on the electrode/counter-electrode surface that facilitates enzyme attachment or the growth of microbial cells. DE 10 2006 054 954 B4 teaches a method for the one-step purification of an enzyme by reversible immobilisation of the enzyme on a modified electrode with subsequent electron transfer between this electrode and the enzyme via a conductive polymer such as polythiophene, polypyrrol or polyanaline for electrochemically controlled biocatalysis. Finally, the electrodes and/or counter-electrodes may be coated with a redox polymer. Such redox polymers are characterized by a nonconductive backbone to which redox side chains are attached.

In a preferred embodiment, only the electrodes of the first, i.e., the lower, support plate are coated. This simplifies introduction of the electrodes into the wells without harming the coating.

Alternatively, however, it is also possible to coat only the counter electrodes of the second support plate, while the electrodes of the lower support plate remain uncoated. It is also possible to coat both the electrodes of the first support plate and the counter-electrodes of the second support plate. In addition, only a subset of the electrodes and/or counter-electrodes on the respective support plates can be coated. In alternative embodiments, the plurality of electrodes and/or counter electrodes are not coated. For example, the bioelectrocatalysts and/or or other inorganic, organic or mixed organic/inorganic materials may alternatively be present inside the electrically conductive solution rather than being immobilized on the electrodes and/or counter-electrodes. Likewise, coating of the electrodes may be omitted if the device according to the invention is to be used only for the analysis of purely electrochemical reactions that occur independently of bioelectrocatalysts.

The invention further provides a method for cell-individual electrical and/or gas-analytical evaluation of at least one electrochemical reaction using the device according to the invention, wherein the method comprises steps of:
i) inserting a microtiter plate into the base element,
ii) filling at least one well of the microtiter plate with an electrically conductive solution,
iii) optionally, coating at least one of the plurality of electrodes and/or one of the plurality of counter-electrodes with an enzyme and/or a cell and/or another inorganic, organic or mixed organic/inorganic material, preferably an enzyme,
iv) assembling the device;
v) carrying out at least one electrochemical reaction, optionally at least one bioelectrochemical reaction, in the device according to the invention; and
vi) evaluating the voltage and current curve of the electrolysis for each cell of the microtiter plate and, optionally, analyzing the composition of gases produced during the at least one reaction.

In step ii) at least one well of the microtiter plate is filled with an electrically conductive solution. The electrically conductive solution is preferably an aqueous solution in which at least one electrolyte is present in dissolved form. By the term electrolyte, the skilled person understands a chemical compound which is dissociated into ions in the solid, liquid or dissolved state and which moves in a directed manner under the influence of an electric field. The electrically conductive solution can thus be an aqueous solution containing ions, e.g., Na⁺- and Cl⁻- ions. However, the electrically conductive liquid does not have to be water-based; in another embodiment, the electrically conductive solution can also be a molten salt or an ionic liquid. The electronically conductive solution may comprise at least one educt of the reaction that is carried out. Alternatively, the educt or educts may be added later, before the device is assembled, or may be led into the wells of the microtiter plate via a ventilation duct. The electrically conductive solution may also be a medium suitable for culturing bacteria or eukaryotic cells, e.g., a cell culture medium. In this case, the bacteria or other cells may also produce at least one educt of the invention.

The steps i-iii) of the methods according to the invention can be carried out in any order. Preferably, the microtiter plate is first inserted into the base element, before at least one of its wells is filled with an electrically conductive solution. However, it is equally possible to first load the at least one well of the microtiter plate with the electrically conductive solution before inserting the microtiter plate into the base element.

Optional step iii) can be achieved in multiple different ways. According to one embodiment, each electrode and/or counter-electrode is treated or coated individually, e.g., with one or more suitable enzymes, biological cells or other inorganic, organic or mixed organic/inorganic materials, which, optionally may possess catalytic activity, as described herein. In addition, the individual electrodes or the individual counter-electrodes can be coated either completely or only partially, e.g. one-sided or two-sided. The treatment of the electrodes and/or counter-electrodes may be carried out either before or after the electrodes and/or counter-electrodes have been fixed to the first or second support plates. Furthermore, it is possible to coat the entire plurality of electrodes and/or the entire plurality of counter-electrodes simultaneously, for example by first fixing the electrodes and/or the counter-electrodes to the first and/or second support plates as described herein before dipping the lower portion of the plurality of electrodes and/or the plurality of counter-electrodes into a bath comprising, for example, a suitable enzyme or a suitable biological cell.

Assembling the device according to invention in step iv) may be done as described herein. For instance, after filling at least one well of a microtiter plate with an electrically conductive solution and inserting the microtiter plate into the base element, the first support plate carrying the plurality of, optionally, pre-treated and/or coated electrodes is arranged on top of the base element accommodating the microtiter plate. For this, the first support plate is positioned above the base element in such a way that the lower portion of at least one electrode of the plurality of electrodes is immersed into the electrically conductive solution that was filled into at least one of the wells of the microtiter plate. The second support plate, which carries the plurality of, optionally pre-treated or coated, counter-electrodes, is next arranged above the first support plate in such a way that the lower portion of at least one counter-electrode passes through a corresponding opening in the first support plate, immediately adjacent to the at least one electrode, so that the at least one electrode and the at least one counter-electrode are immersed in the electrically conductive solution of the same well of the microtiter plate, as described herein. For carrying out the at least one bioelectrochemical reaction in step v), a current is applied by the CU to the at least one electrode and the at least one counter-electrode which then begin to electrically communicate with each other through the electrically conductive solution.

Step vi) is performed by the CU, which receives signals transmitted by the different sensors described herein. The analysis and evaluation preferably takes place while the reaction in the corresponding cell of the microtiter plate is on-going. This way, the CU is able to adjust one or more reaction parameters while running the reaction, to modulate the outcome of the reaction.

The device according to the invention described herein and the associated method according to the invention make it possible to analyze and evaluate a plurality of bioelectrochemical and/or bioelectrochemical reactions running in parallel on a cell-individual basis. The analysis and evaluation is thus not limited to a single electrochemical or bioelectrochemical reaction.

Further features and advantages of the present invention will be apparent from the following description with reference to the drawings.
- Fig. 1: shows an exploded view of a first embodiment the device according to the invention.
- Fig. 2: shows an isometric view of the first and the second support plates of the device of Fig. 1.
- Fig. 3: shows an isometric view of the assembled device of Fig. 1.
- Fig. 4: shows an isometric view of the first support plate and the second support plate of Fig. 2 without electrodes and counter-electrodes, respectively.
- Fig. 5: shows an exploded view of a second embodiment of the device according to the invention.
- Fig. 6: shows an isometric view of the assembled device of Fig. 5.
- Fig. 7: shows schematically a single electrolysis set-up of the assembled device of Fig. 5.
- Fig. 8: shows a schematic example of how an electrode can be electrically contacted in the assembled device of Fig. 1 or 5.
- Fig. 9: Comparison of 4 different sensor types capable of sensing H₂ in a gas-stream

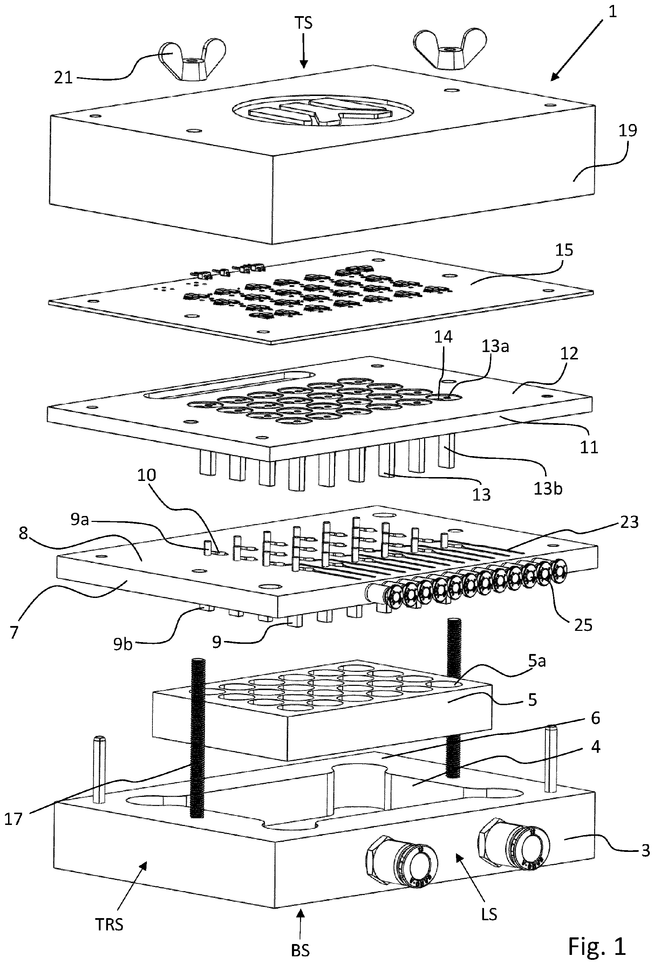

Fig. 1 shows an exploded view of a first embodiment of the device 1 according to the invention. The device 1 preferably has a rectangular shape in plan view and has a top side TS, a bottom side BS opposite the top side TS, two opposite longitudinal sides LS and two opposite transverse sides TRS. The device 1 according to the invention has a modular structure, i.e. it includes different elements or modules that can be operably arranged one above the other.

The base element 3 defines the bottommost element or module of the device 1. As can be seen in **Fig. 1****,** the base element comprised a recess 4 on its upper surface 6 that provides sufficient space to accommodate and hold a conventional, commercially available microtiter plate 5 comprising a plurality of wells 5a, preferably with a precise fit. Other designs for accommodating and/or fixating the microtiter plate 5 to the base element 3 are conceivable and are discussed in the specification above.

The base element 3 shown in **Fig. 1** further comprises at least two screws 17 positioned on opposite sides of the upper surface 6, wherein the threads of the screws 17 project upwards perpendicularly to the upper surface 6 of the base element 3. The screws 17 facilitate the stacking of the other elements and thus the assembly and tight closure of the device 1. However, the screws 17 merely constitute an optional embodiment of the invention. The stability of the device 1 can be further enhanced by pins that can extend through one or more of the elements of the device 1. The individual elements of the device 1 according to the invention can also be held together and stabilized by other means, for example by using clamps as described herein or other mechanical or magnetic connection means.

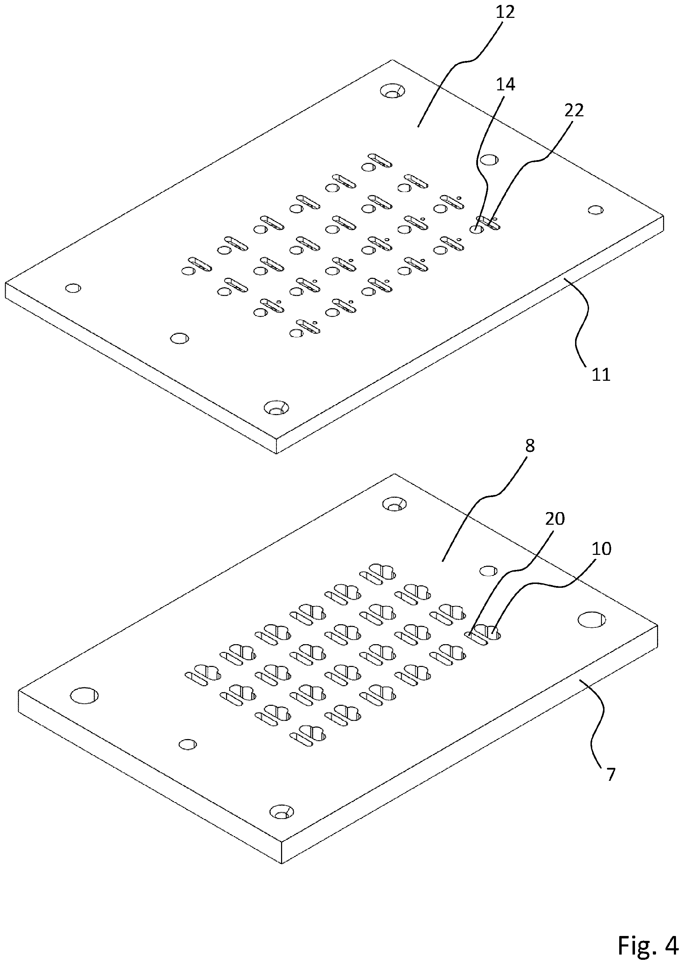

A first support plate 7 is stacked on top of the base element 3 comprising the microtiter plate 5, e.g., by passing the threads of the at least two screws 17 through corresponding through-holes drilled through the first support plate 7. The first support plate 7 comprises a plurality of solid, elongated electrodes 9 that correspond in number and arrangement to the wells 5a of the microtiter plate 5. When the device 1 is fully assembled, each electrode 9 of the first support plate extends into a corresponding well 5a of the microtiter plate 5 so that it can be immersed into an electrically conductive solution that has been filled into the wells 5a of the microtiter plate 5 prior to assembly of the device 1. As can be seen in **Fig. 1** and in more detail, in **Fig. 2** and **4****,** the plurality of electrodes 9 is essentially perpendicularly attached to the first support plate 7 by being inserted into a corresponding plurality of holes 20 extending through the entire depth of the first support plate 7. Each electrode 9 comprises a lower portion 9b which extends downwards from the first support plate 7 into the wells 5a of the microtiter plate 5 as well as an upper portion 9a which penetrates through a corresponding hole 20 inside the first support plate 7 and protrudes upwards out of the upper surface 8 of the first support plate 7. The first support plate 7 further comprises a plurality of through-holes 10, each through-holes 10 being adjacent to each hole 20 carrying an electrode 9 for receiving the lower portion 13b of a corresponding counter-electrode 13 that is attached to a second support plate 11.

The first support plate 7 is preferably also configured to seal the individual wells 5a of the microtiter plate 5. Accordingly, the underside of the first support plate 7 may comprise a sealing layer (not shown), e.g. of rubber or latex, which presses onto the microtiter plate 5.

When assembling the device 1 according to the shown embodiment, the second support plate 11 comprising a plurality of elongated counter-electrodes 13 is stacked on top of the first support plate 7, e.g., by passing the threads of the at least two screws 17 through corresponding holes 22 drilled through the second support plate 11. In the embodiment, the plurality of counter-electrodes 13 corresponds in number and arrangement both to the number and arrangement of the plurality of electrodes 9 of the first support plate 7 as well as the number and arrangement of the plurality of wells 5a of the microtiter plate 5. As can be seen in **Fig. 1** and in more detail, in **Fig. 2** and **4****,** the plurality of counter-electrodes 13 is attached to the second support plate 11 by having their upper portion 13a inserted into a corresponding plurality of holes 22 extending through the entire thickness of the second support plate 11. When the device 1 is fully assembled, the counter-electrodes 13 are positioned directly above the through-holes 10 of the first support plate 7. Each counter-electrode 13 comprises a lower portion 13b which then extends through a corresponding through-hole 10 in the first support plate 7 into the wells 5a of the microtiter plate 5 below. In doing so, each counter-electrode 13 of the second support plate 11 combines with a corresponding electrode 9 of the first support plate 7 to form a pair of electrode and counter-electrode inside a common well 5a of the microtiter plate 5. The electrode 9 and the counter-electrode 13 of such a pair are not in direct contact with each other but run in parallel or nearly in parallel while being separated by a gap. A pair of electrode 9 and counter-electrode 13 only electrically communicates when being in contact with an electrically conductive solution inside an individual well 5a of the microtiter plate 5. The second support plate 11 further comprises a through-hole14 adjacent to each hole 22 carrying a counter-electrode 13 for receiving the upper portion 9a of a corresponding electrode 9 (see **Figs. 1****,** **2** **and** **4****).** As can be deduced from **Fig. 1** and **2****,** the upper portions of both the plurality of electrodes 9 and the plurality of counter-electrodes 13 are freely accessible on the upper surface 12 of the second support plate 11 and can thus be electrically contacted.

**Fig. 4** shows the first support plate 7 and the second support plate 11 without electrodes 9 or counter-electrodes 13. The first support plate 7 comprises a plurality of holes 20 into which the plurality of electrodes 9 can be inserted. Adjacent to each hole 20 is an opening 10 for receiving the lower portion 13b of a counter-electrode fixed to the second support plate 11 in the assembled device (1), i.e. when the first support plate 7 is engaged with the second support plate 11. The second support plate 11 comprises a plurality of holes 22 into which the plurality of counter-electrodes 13 can be inserted. Adjacent to each hole 22 is a through-hole 14 for receiving the upper portion 9a of an electrode 9 protruding from the upper surface 8 of the first support plate 7 in the assembled device (1).

As can also be seen from **Fig. 1** and **Fig. 2****,** the lower portions of the electrodes 9 or counter-electrodes 13, which are to project into the wells 5a of the microtiter plate 5, can be comparatively wider than their upper portions, which are fixed to the first and second support plates 7, 11, respectively. However, this particular shape of the electrodes 9 and/or the counter-electrodes 13 is not necessarily required. It is equally possible that the electrodes 9 and counter-electrodes 13 are, e.g., solid rods or elongated plates of uniform thickness.

The plurality of electrodes 9 and/or the plurality of counter-electrodes 13 are preferably coated with a bioelectrocatalyst, e.g. an enzyme or a biological cell, as described herein.

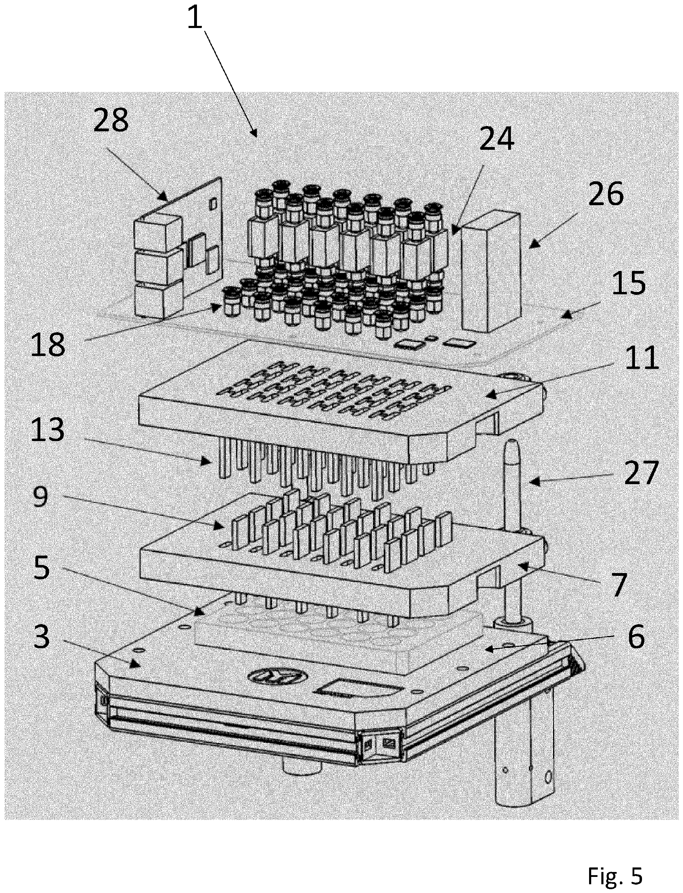

As shown in **Fig. 1****,** the plurality of electrodes 9 and the plurality of counter-electrodes 13 are electrically contacted by a printed circuit board (PCB) 15. The PCB 15 forms an electrical connection to each pair of electrode 9 and counter-electrode 13 to facilitate electrolysis in each individual cell of the microtiter plate 5. Therefore the PCB 15 is operably connected to a suitable power source (not shown) and may comprise electrical lines or electronic circuitry. The PCB is further connected to a suitable control unit (CU, not shown), which may be part of an external computer system or may, as shown in Fig. 5, form an integral part of the device (1), e.g., by being configured as a single-board computer (SBC), in particular a microcontroller unit (MCU) directly mounted to the PCB. The PCB 15 may comprise various electronic sensors (not shown), e.g., current sensors for monitoring current-consumption, potential sensors for monitoring the applied potential as well as gas sensors for detecting reaction gases for each cell of the microtiter plate 5. As can be seen in **Fig. 1****,** the PCB 15 can be stacked on top of the second support plate 11, e.g., by passing the threads of the at least two screws 17 through corresponding holes drilled through the PCB 15. The PCB 15 is configured to electrically contact the upper surface of every electrode 9 and every counter-electrode 13 individually.

Even though the PCB 15 in **Fig. 1** is arranged above the second support plate 13, this arrangement is not required. It is also possible to mount the PCB 15 e.g. on one side of the device 1, whereby the individual electrodes 9 and counter-electrodes 13 can be brought into contact with the PCB 15 via wires or cables or other circuitry, as described herein.

To protect the sensitive electronic components on the PCB 15 as well as the electrodes 9, 13 from damage and/or contamination, the device 1 may be covered by a lid 19. Like the other elements before, the lid 19 can be stacked on top of the other elements of the device 1 by passing the threads of the at least two screws 17 through corresponding holes drilled through the lid 19.

To fix the lid 19 firmly on the device 1, nuts 21 can be screwed tightly onto the screws 17 protruding through the upper surface of the lid 19. The fully assembled device 1 is depicted in **Fig. 3****.** As can be seen, the first and second support plates 7, 11 are clamped between the lid 19 and the base element 3 in the assembled device. However, it is also possible that the base element 3 and the two support plates 7, 11 are completely covered by the lid 19. It should also be noted that other ways of fixing the lid 19 onto base plate 3 are possible, e.g. by a mechanical or magnetic snap-fit connection. The essence of the connection is a reliable connection which may be released and opened in a suitable manner so that the components may be quickly separated, processed, interchanged and reassembled.

As can be seen in **Fig. 1-3****,** the first support plate 7 can further comprise a plurality of ventilation ducts 23 that allow for the transport of air to each individual well 5a of the microtiter plate 5 inserted into the base element 3. The ventilation ducts 23 may preferably extend from the inside of each individual well 5a of the microtiter plate 5 though at least the first support plate 7 where they arrive at a plurality of sealable outlets 25 located, e.g., at a side surface of the first support plate 7. In alternative embodiments, the air may also be guided through ventilation ducts 23 that extend as well through the second support plate 11 and, optionally, also the PCB 15 and/or the lid 19 before leaving the device 1 according to the invention via outlets 25. In some embodiments, the ventilation ducts 23 may also be used to transport reaction gases out of the wells 5a of the microtiter plate 5. In such a scenario, the outlets 25 may optionally be suitably connected, e.g., via tubing to an external gas analyzer (or gas sensor, not shown) which is able to determine the exact identity and composition of the gases produced during the bioelectrochemical reactions in each individual well 5a.

Fig. 5 depicts an exploded view of a preferred embodiment of the device according to the invention. The device corresponds essentially to the setup described in the first embodiment.

Like the device 1 of the first embodiment, the device 1 of the preferred embodiment preferably has an essentially rectangular shape in plan view and comprises a base element 3 having an upper surface 6 for accommodating a commercially available microtiter plate 5, a first support plate 7 comprising a plurality of electrodes 9 that extend into the wells of the microtiter plate 5, a second support plate 11 comprising a plurality of counter-electrodes 13 extending through corresponding through-holes in the first support plate 7 into the wells of the microtiter plate 5 and a PCB 15 making electrical contact with both the electrodes 9 and the counter-electrodes 13. As described above, the first support plate 7 may comprise a sealing layer (not shown), e.g., of rubber or latex, which presses onto the microtiter plate 5 to seal the individual wells. However, the sealing layer may also be provided as a separate component or module (not shown) of the device 1 to separate the microtiter plate 5 from the overlying support plates 7, 11. In this case, the sealing layer may be, e.g., a separate mat or sheet made of a rubber material which comprises a plurality of holes arranged to allow, e.g., the electrodes 9 and counter electrodes 13 to pass through the sealing layer into each respective well.

Fig. 5 further shows a plurality of gas ducts 18 that are connected via additional through-holes in the PCB 15 and the first and second support plates 7, 11 to the wells of the microtiter plate 5. The gas ducts 18 enable the transport of various reaction gases from every individual cell of the microtiter plate 5 to gas sensors 24 mounted onto the PCB 15. The gas sensors 24 may be, e.g., Micro-Electro-Mechanical Systems (MEMS) sensors, chemiresistors, solid electrolyte sensors, nondispersive infrared (NDIR) sensors or various combinations thereof. The reaction gases are aspired from the wells of the microtiter plate 5 via the activity of a gas pump 26, which may be mounted onto the PCB 15 as well.

The gas sensors 24 are connected to a CU 28, which may be configured as a single-board computer (SBC) or, as shown in Fig. 5, as a microcontroller unit (MCU) directly mounted to the PCB 15. The CU 28 may however also be part of an external computer system, which is not installed in the device according to the invention. The CU 28 is further connected to the gas pump 26 to control its activity and thus the amount of gas that passes the gas sensors 24. To avoid the development of a vacuum inside the wells of the microtiter plate 5 upon aspiration of the reaction gases and to enable a uniform gas flow through the gas ducts 18 past the gas sensors 24, each individual cell of the microtiter plate 5 is further connected to a ventilation duct (not shown) through which a constant flow of air can be drawn in from the environment by the gas pump 26. Albeit not depicted in Fig. 5, the ventilation ducts may be configured like the ventilation ducts 23 shown in Figs. 1-3. Likewise, the ventilation ducts may, in some embodiments, be used to transport reaction gases out of the wells of the microtiter plate 5 to outlets that are suitably connected via tubing to an external gas analyzer (not shown) for additional gas analysis, e.g., to further supplement the measurement results of the gas sensors 24.

Alternatively, external gas-sensors can be connected to a plurality of individual cells, e.g., via the gas ducts, via tubing.

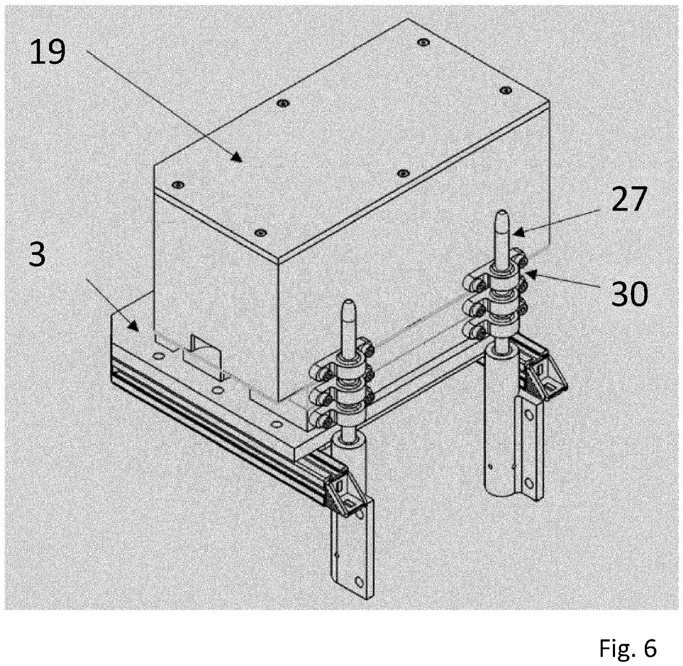

As seen in Fig. 5 and 6, the base element 3 may comprise guide rods 27 projecting upwards perpendicularly to the upper surface 6 of the base element 3 rather than screws 17 for stacking the of the other elements and thus the assembly and tight closure of the device 1. To do so, at least the first and the second support plates 7, 11 and the lid 19 are equipped with guides 30 such as pillow block bearings through which the guide rods 27 can be passed.

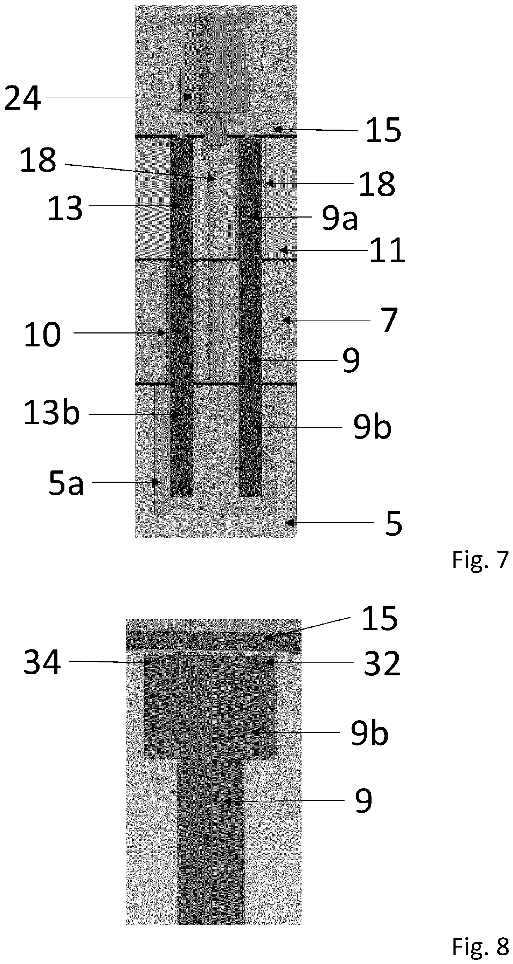

Fig. 7 shows schematically a single electrolysis set-up of the assembled device 1. In each well 5a of a microtiter plate 5 a separate electrolysis reaction takes place. For this, a first support plate 7, to which an electrode 9, e.g. a cathode, is attached, is arranged above the microtiter plate 5 in such a way that a lower section 9b of the electrode 9 projects into the well 5a of the microtiter plate 5. Above the first support plate 7, a second support plate 11 is arranged to which a counter-electrode 13, e.g., an anode, is attached. A lower section 13b of the counter-electrode 13 extends through a through-hole 10 of the first support plate 7 adjacent to the electrode 9 into the well 5a of the microtiter plate 5.

Meanwhile, an upper section 9a of the electrode 9 extends through a corresponding through-hole 14 in the second support plate 11, so that both the electrode 9 and the counter-electrode 13 can be electrically contacted at the upper surface of second support plate 11 via a PCB 15. Adjacent to the electrode 9 and the counter electrode 13, a gas duct 18 extends from the well 5a of the microtiter plate 5 through the first and second support plates 7, 11, optionally via additional through-holes (not shown) in these plates and is connected to a gas sensor 24 mounted on the PCB 15.

Fig. 8 shows a schematic example of how an electrode 9 can be electrically contacted in the device 1 according to the invention. For this purpose, two spring contacts 32, 34 are soldered to the bottom side of the PCB 15, which contact the upper section 9a of the electrode 9 and thus enable a stable and uniform current flow through the electrode 9.Double contacting of the electrodes as shown in Fig. 8 establishes a more stable connection than single contacting and thus compensates for potential assembly inaccuracies and reduces contact resistances.

### Example

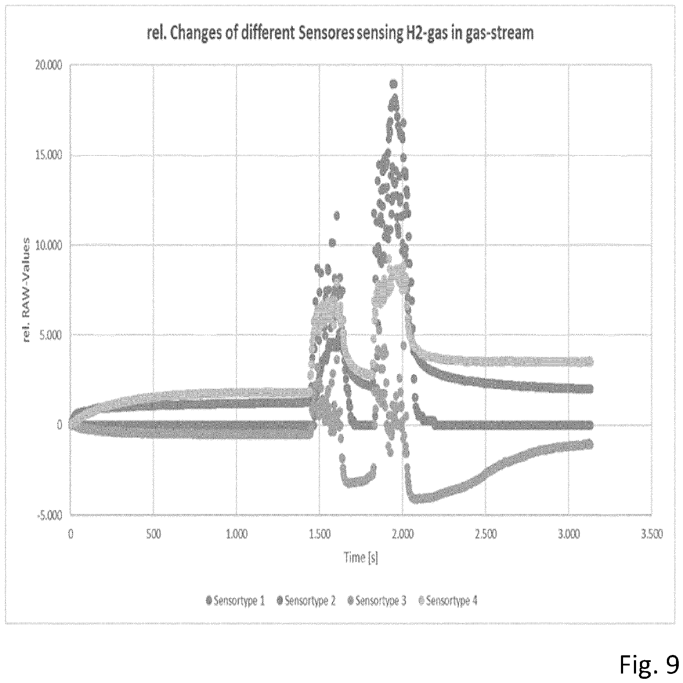

Using the device according to the invention, the inventors tested four different types of gas sensors in a proof-of-principle experiment for the detection of H₂ gas within a gas stream. The production of H₂ gas is considered an indicator of small amount side-reaction progress. The experiment showed that the four different sensors could detect H₂ gas with different sensitivity. The selection of suitable sensors depends in particular on whether they produce minimum signal noise under different operating conditions (e.g. different electrolysis conditions). In the present example, sensor types 2 and 4 showed comparatively low scattering and would thus be preferred (Fig. 9).

The experiment illustrates that a device according to the invention equipped with suitable gas sensors allows for cell-individual gas analyses. Additional gas sensors can be used, for example, to detect CO₂, alcohols and nitrogen species in gas mixtures.

## Claims

1. A device (1) for cell-individual electrical and gas-analytical evaluation of at least one electrochemical reaction, comprising:
a) a base element (3) suitable for accommodating a microtiter plate (5) comprising a plurality of wells (5a), wherein each well (5a) defines a distinct individual cell when being filled with an electrically conductive solution;
b) a first support plate (7) on top of the base element (3), wherein the first support plate (7) comprises a plurality of electrodes (9) corresponding to the number and arrangement of the plurality of wells (5a) of the microtiter plate (5), wherein the plurality of electrodes (9) is fixed to the first support plate (7) in a manner that allows for electrically contacting the electrodes (9), wherein each electrode (9) is configured to extend from the first support plate (7) into the electrically conductive solution in a corresponding well (5a) in said microtiter plate (5), wherein the first support plate further comprises a plurality of through-holes (10), each through-hole (10) being adjacent to each electrode (9);
c) a second support plate (11) on top of said first support plate (7), wherein said second support plate (11) comprises a plurality of counter-electrodes (13), wherein the number and arrangement of the plurality of counter-electrodes (13) corresponds to the number and arrangement of the plurality of electrodes (9) of the first support plate (7), wherein the plurality of counter-electrodes (13) is fixed to the second support plate (11) in a manner that allows for electrically contacting the counter-electrodes (13) and wherein each counter-electrode (3) is configured to extend from the second support plate (11) through a corresponding through-hole (10) in the first support plate (7) to run adjacent to a corresponding electrode (9) into the electrically conductive solution of a corresponding well (5a) in said microtiter plate (5);
d) one or more printed circuit boards (PCBs) (15) electrically contacting each electrode of the plurality of electrodes (9) and each counter-electrode of the plurality of counter-electrodes (13);
e) a gas pump (26) configured to transport reaction gases generated during the at least one electrochemical reaction from the respective wells (5a) of the microtiter plate (5) through a plurality of gas ducts (18) to a plurality of internal or external gas sensors (24), wherein each individual well (5a) of the microtiter plate 5 is further connected to a ventilation duct (23) through which air can be drawn from the environment (26); and
f) a lid (19),
wherein the configuration of each pair of electrode (9) and counter-electrode (13) extending into each well (5b) allows for electrochemical communication inside the individual cell.

2. The device (1) of claim 1, further comprising a control unit (CU) (28) electrically connected to the one or more PCBs (15), wherein the CU (28) is configured to receive and analyze data from the at least one electrochemical reaction in each well (5a) of the microtiter plate (5) by being operably connected to the plurality of gas sensors (24) as well as to sensors for monitoring current-consumption and the applied potential in each individual cell.

3. The device (1) of any of the preceding claims, wherein the CU (28) is part of a single-board-computer (SBC), preferably, a microcontroller unit (MCU) mounted to the one or more PCBs (15).

4. The device (1) of any of claims 2 to 3, wherein each electrode of the plurality of electrodes (9) and each counter-electrode of the plurality of counter-electrodes (13) are configured to be individually controlled by the CU (28) during use of the device (1).

5. The device (1) of any of claims 1 to 4, wherein the plurality of electrodes (9) and/or the plurality of counter-electrodes (13) are coated with an enzyme, a biological cell and/or another inorganic, organic or mixed organic/inorganic material, preferably an enzyme.

6. The device (1) of any of the preceding claims, comprising a plurality of internal gas sensors (24) selected from the group comprising Micro-Electro-Mechanical Systems (MEMS) sensors, chemiresistors, solid electrolyte sensors, nondispersive infrared (NDIR) sensors and combinations thereof.

7. The device (1) of any of the preceding claims, wherein the electrodes (9) and counter-electrodes (13) are exchangeable and/or reusable.

8. The device (1) of any of the preceding claims, wherein the plurality of electrodes (9) and the plurality of counter-electrodes (13) comprise a material selected from the group comprising carbon, glassy carbon, copper, lead, zinc, tin, platinum, silver, gold, stainless steel, iron, metal alloys, conductive ceramics and conductive polymers.

9. The device (1) of any of the preceding claims, wherein the plurality of electrodes (9) and the plurality of counter-electrodes (13) consist of the same material.

10. The device (1) of any of claims 1-8, wherein the plurality of electrodes (9) consists of a different material than the plurality of counter-electrodes (13).

11. The device (1) of any of the preceding claims, wherein the one or more PCBs (15) are arranged on top of the second support plate (11).

12. The device (1) of any of the preceding claims, further comprising a third support plate (40), preferably located between the second support plate (11) and the PCB (15), wherein the third support plate (40) comprises a plurality of reference electrodes (42) corresponding to the number and arrangement of the plurality of electrodes (9) of the first support plate (7) and the plurality of counter-electrodes (13) of the second support plate (11), wherein each reference electrode (42) is configured to extend from the third support plate (40) through corresponding through-holes in the first and the second support plates (7, 11) adjacent to a corresponding electrode (9) and a corresponding counter-electrode (13) into the electrically conductive solution in a corresponding well (5a) in said microtiter plate (5), wherein the third support plate further comprises a plurality of through-holes (44) for allowing passage of the plurality of electrodes (9) and the plurality of counter-electrodes (13), so that the plurality of electrodes (9) and the plurality of counter-electrodes (13) can be electrically contacted, wherein each through-hole (44) is adjacent to each reference electrode (42);

13. A method of preparing a product of at least one electrochemical reaction using the device (1) of any one of claims 1-12, wherein the method comprises steps of:
i) inserting a microtiter plate (5) into the base element (3) of the device (1),
ii) filling at least one well (5a) of the microtiter plate (5) with an electrically conductive solution,
iii) optionally, coating at least one of the plurality of electrodes (9) and/or one of the plurality of counter-electrodes (13) with an enzyme and/or a cell and/or another inorganic, organic or mixed organic/inorganic material, preferably an enzyme,
iv) assembling the device (1);
v) carrying out at least one electrochemical reaction having at least one product, optionally at least one bioelectrochemical reaction, in the device (1) of any one of claims 1-13,
vi) optionally, isolating the at least one product of at least one electrochemical reaction.

14. A method of cell-individual electrical and/or gas-analytical evaluation of at least one electrochemical reaction, optionally at least one bioelectrochemical reaction, using the device (1) of any one of claims 1-12, wherein the method comprises steps of:
i) inserting a microtiter plate (5) into the base element (3) of the device (1),
ii) filling at least one well (5a) of the microtiter plate (5) with an electrically conductive solution,
iii) optionally, coating at least one of the plurality of electrodes (9) and/or one of the plurality of counter-electrodes (13) with an enzyme and/or a cell and/or another inorganic, organic or mixed organic/inorganic material, preferably an enzyme,
iv) assembling the device (1);
v) carrying out at least one electrochemical reaction, optionally at least one bioelectrochemical reaction, in the device (1) of any one of claims 1-12; and
vi) evaluating the voltage and current curve of the electrolysis for each cell of the microtiter plate (5) and, optionally, analyzing the composition of gases produced during the at least one electrochemical reaction, optionally bioelectrochemical reaction.

15. The device (1) of any of claims 1-12 and the methods of any of claims 13 or 14, wherein a plurality of electrochemical reactions and/or bioelectrochemical reactions, running simultaneously, are evaluated.
